(19) Europäisches Patentamt
European Patent Office
Office européen des brevets

(11) Publication number: **0 117 146**
**B1**

(12) **EUROPEAN PATENT SPECIFICATION**

(45) Date of publication of patent specification: **30.12.86**

(21) Application number: **84301079.4**

(22) Date of filing: **20.02.84**

(51) Int. Cl.$^4$: **C 07 C 51/215,** C 07 C 51/25, C 07 C 57/04, C 07 C 5/333, C 07 C 11/06, C 07 C 45/35, C 07 C 47/22

# (54) Conversion of propane to acrylic acid.

(30) Priority: **22.02.83 US 468527**

(43) Date of publication of application:
**29.08.84 Bulletin 84/35**

(45) Publication of the grant of the patent:
**30.12.86 Bulletin 86/52**

(84) Designated Contracting States:
**BE DE FR GB IT NL**

(56) References cited:
**GB-A-2 118 939**

(73) Proprietor: **THE HALCON SD GROUP, INC.**
**2 Park Avenue**
**New York, N.Y. 10016 (US)**

(72) Inventor: **Khoobiar, Sargis**
**750 Ridge Road**
**Kinnelon New Jersey 07405 (US)**
Inventor: **Porcelli, Richard V.**
**287 Crestwood Avenue**
**Yonkers New York 10707 (US)**

(74) Representative: **Cropp, John Anthony David et al**
**MATHYS & SQUIRE 10 Fleet Street**
**London, EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 117 146 B1

**Description**

The invention relates generally to the preparation of acrylic acid. More specifically the invention relates to the preparation of acrylic acid in a three-stage process from propane.

In typical processes of the prior art propylene is reacted in the vapor phase with molecular oxygen over a catalyst to produce acrolein. The reactor effluent containing acrolein is then passed to a second oxidation step where it is reacted with molecular oxygen in the vapor phase over a different catalyst to form acrylic acid. Generally, the feed for this process has been the unsaturated olefin. Any saturated hydrocarbon present was considered to be essentially an inert since little, if any, oxidation occurred. Known dehydrogenation processes could be used to prepare propylene from propane as a feedstock for the known methods of preparing acrylic acid. However, if propylene must be purified before oxidation, then this would be an uneconomic way of preparing acrolein and acrylic acid.

Integrated processes are not generally used because the by-products of dehydrogenation cannot be present in the subsequent use of propylene, without causing contamination of the ultimate end product. The present process relates to an integrated process in which the dehydrogenation of propane is combined with the oxidation of the resulting propylene to acrylic acid without intermediate separation and purification of the propylene before the oxidation is carried out.

In British Patent 1,340,891 propane and oxygen are reacted to prepare propylene, acrolein, and acrylic acid over a variety of base metal oxide catalysts. Since the conversion of propane is quite low, high concentrations of propane are used so that the net amount of propylene produced is sufficient to result in a practical process.

U.S. Patent 4,260,822 also discloses a process for direct oxidation of propane to acrylic acid in a single step, again using large amounts of propane in order to overcome the relatively low conversion of propane to the product.

The above one-step processes are not economic, since the conversions are quite low and require recycling of substantial amounts of unreacted feed in order to convert a high proportion of the propane fed. Also, the catalysts typically do not have the long useful life necessary for satisfactory commercial operations.

A number of processes have been developed for converting propane to propylene. In U.S. Patent 3,784,483 a cobalt, iron, and phosphorus catalyst is disclosed as useful for the oxidative dehydrogenation of propane to propylene.

In a group of patents exemplified by U.S. 4,083,883 a precious metal combined with promoter metals on a support is used for the dehydrogenation of paraffins, particularly normal paraffins.

Another approach is taken in U.S. Patents 3,692,701, 4,005,985 and 4,041,099. In these processes large quantities of steam are used to dehydrogenate propane over a catalyst of platinum-tin on zinc aluminate. Dehydrogenation of propane is shown over zinc titanate catalysts in U.S. 4,144,277 and 4,176,140.

Many patents have disclosed processes for oxidation of propylene to acrylic acid, such as EP 0000663 and U.S. 3,954,855. These processes are generally carried out in two steps in order to obtain the best yields of acrylic acid. In the first step propylene is oxidized to acrolein in the vapor-phase over a promoted molybdenum catalyst and the effluent from the reaction is passed with added oxygen over a second promoted molybdenum catalyst to produce acrylic acid.

Based on the prior art discussed above, one might assemble a multi-step process wherein propane is dehydrogenated with or without the presence of oxygen to produce propylene, which would be separated and purified and fed to a conventional acrylic acid process. In this way, substantially all of the propane fed is converted to acrylic acid by merely combining known processes. However, this may not be an economic way to produce acrylic acid. As will be seen, the present invention pertains to an integrated process by which propane may be converted to acrylic acid without first purifying an intermediate propylene product.

Dehydrogenation of propane produces substantial amounts of hydrogen and small amounts of lower molecular weight hydrocarbons, which in the prior art processes would be removed from the product, but which in an integrated process must be accommodated in the oxidation of propylene to acrolein. The hydrogen and by-products should not have an adverse effect on the oxidation catalysts or their performance. For example, the exposure of the by-products to oxidizing conditions cannot produce contaminants which reduce the quality of the acrylic acid. Also, the presence of hydrogen should not create an explosive mixture in the oxidation reactors.

According to the present invention there is provided a process for the preparation of acrylic acid from propane comprising:

(a) in a first stage dehydrogenating propane to propylene, optionally in the presence of steam, over a dehydrogenation catalyst to form an effluent stream comprising propylene, hydrogen, carbon oxides and unreacted propane;

(b) in a second stage mixing oxygen and optionally steam with said effluent stream of (a) and passing the mixture over a first oxidation catalyst at conditions selected to produce a second effluent stream comprising acrolein, unreacted propylene and propane, and oxygen, hydrogen and carbon oxides;

(c) in a third stage passing the effluent stream of (b) optionally with additional oxygen over a second oxidation catalyst at conditions selected to produce a third effluent stream comprising acrylic acid, unreacted propylene, propane and acrolein, and oxygen, hydrogen and carbon oxides;

(d) recovering the acrylic acid from said effluent of (c) to produce an acrylic acid-depleted effluent; and
(e) returning at least the unreacted propane from said acrylic acid-depleted effluent to step (a).

In the process of the invention, acrylic acid is prepared from propane in an integrated process in which dehydrogenation of propane to propylene in a first stage is followed immediately, that is, without separation and purification of the propylene from the dehydrogenation effluent, by addition of oxygen and oxidation of the propylene to acrolein in a second stage. Alternatively, the dehydrogenation effluent may be cooled and water condensed and separated before the effluent stream enters the oxidation stage. The oxidation stage may be followed by immediate oxidation of the acrolein in the second stage effluent to acrylic acid in a third stage. Alternatively, the acrolein may be recovered or concentrated in the second stage effluent before being fed to the third stage for oxidation.

The acrylic acid product is recovered by scrubbing or quenching the third stage effluent which also contains unreacted propane, propylene, and oxygen, plus hydrogen, carbon oxides and miscellaneous light and heavy hydrocarbon by-products of the dehydrogenation and oxidation reactions. Acrylic acid may be absorbed in water to separate it from the lighter by-products and unreacted feed components. Thereafter, at least unreacted propane is recycled. Residual gases depleted in acrylic acid may be scrubbed to recover propane and propylene, which are recycled to the first stage for dehydrogenation. Alternatively, a selective oxidation may be employed to remove oxygen from the residual gases and carbon dioxide removed by scrubbing with a suitable solvent.

The dehydrogenation of propane to propylene is carried out by a vapor-phase reaction over a suitable catalyst, which may be platinum with or without a promoter on a zinc aluminate support or other suitable noble and base metal catalysts. When the catalyst is platinum, with or without a promoter, on zinc aluminate the dehydrogenation will be carried out at 400 to 700°C and up to 10 bar (10 kg/cm$^2$) gauge pressure, with a propane to steam ratio of 1/0.5 to 1/10.

The oxidation of propylene to acrolein and of acrolein to acrylic acid may be carried out over suitable catalysts, such as mixed base metal oxides, especially molybdenum oxide-based catalysts.

Description of the drawings

Figure 1 is a block diagram showing the process of the invention.

Figure 2 is a simplified flowsheet showing one embodiment of the invention.

Figure 3 is a simplified flowsheet showing an alternative embodiment of the invention.

Description of the preferred embodiments

In the principal aspect, the invention is an integrated process combining the dehydrogenation of propane to propylene with the oxidation of the propylene to acrylic acid in two steps. The product acrylic acid is separated from the by-products and the unreacted propane may be recovered and recycled to the dehydrogenation step if desired. A schematic view of such a complete process is shown in Figure 1.

The process contrasts with those of the prior art particularly in that the effluent from the dehydrogenation reaction 10 may be fed directly to the oxidation reaction 12 for conversion of propylene to acrolein. One familiar with the prior art would expect that propylene would be separated from the effluent of the dehydrogenation step 10 before feeding it to the oxidation step 12. Since the dehydrogenation of propane involves the production of significant quantities of hydrogen, as well as small amounts of lower molecular weight hydrocarbons, the propylene must be oxidized in the presence of significant quantities of hydrogen, propane, and by-products, without markedly affecting the oxidation of propylene to acrolein or causing oxidation of the hydrogen. We have found that propylene may be oxidized to methacrolein in the presence of hydrogen, propane, and by-products of the dehydrogenation step, while not adversely affecting the oxidation process.

After the propylene has been oxidized to acrolein, the entire effluent from the first oxidation step 12 may be sent directly to the second oxidation step 14 where the acrolein is oxidized to acrylic acid. Thereafter, the effluent of oxidation step 14 is partially condensed and separated at 16. Purified acrylic acid is the product while the lower-boiling components are separated at 18.

Waste gases and heavy by-products are discharged while unreacted propane is recycled to step 10. Alternatively, oxygen and carbon oxides may be removed selectively and the remaining gases recycled. Although acrolein will normally be converted to acrylic acid and by-products, if the conversion is kept at a lower level so that a significant amount of acrolein remains, then acrolein optionally may be recovered and recycled to the oxidation stage 14 as shown.

In another aspect, the effluent from the first oxidation step 12 may be separated to concentrate the acrolein, which is then fed, along with air or oxygen to the second oxidation step 14. Recovery may be made by absorption in suitable liquids, such as water and the lower carboxylic acids. Unreacted propane will be returned to the dehydrogenation step 10 as shown.

Example 1

Dehydrogenation of propane

A catalyst composed of 0.4 wt % Pt and 1 wt % In on a zinc aluminate support was prepared. A catalyst sample of 50 cc as 3 mm extrudate was charged to a tubular reactor having a 25.4 mm i.d. An additional 250 cc of the zinc aluminate support was added to the reactor above the catalyst bed. A gas stream containing 1

mol of propane for each 2 mols of steam was fed to the reactor at a gas hourly space velocity of 4000 $hr^{-1}$ under a pressure of 3.5 bar (3.5 $kg/cm^2$) gauge. The inlet to the catalyst bed was held at a predetermined temperature, with the outlet temperature reflecting the endothermic nature of the dehydrogenation reaction. The results are given below in Table A.

TABLE A

| Test | Inlet temp. °C. | Conversion of propane, % | Selectivity to propylene, % |
|---|---|---|---|
| 1 | 548 | 23.6 | 95.8 |
| 2 | 523 | 29.7 | 96.5 |

The dehydrogenation of propane produces one mol of hydrogen for each mol of propylene and additional hydrogen from the formation of some lower molecular weight by-products. When propylene is not separated before subsequent oxidation the hydrogen and other by-products are carried into the subsequent oxidation reaction. Oxidation of the by-products to compounds which contaminate acrolein must be avoided. Oxidizing the hydrogen along with propylene would be undesirable since it would consume oxygen and interfere with the oxidation of propylene. Also, oxidation of hydrogen would generate undesirable heat and could create hot-spots in the catalyst and lower the production of acrolein. However, it has been found that oxidation of propylene can be carried out in the presence of hydrogen with substantially no consumption of hydrogen, as will be seen in the following example. The presence of propane which has not been dehydrogenated also appears to have no significant effect on the oxidation of propylene.

Example 2

Oxidation of propylene to acrolein

A catalyst was prepared according to the method described in EP 0000663 and corresponding to Reference Catalyst A and having composition of 80%

$$K_{0.1}Ni_{2.5}Co_{4.5}Fe_3Bi_1P_{0.5}Mo_{12}O_x$$

and 20% $SiO_2$. A charge of 220 cc of catalyst as 4.7 mm pellets was placed in a single-tube reactor having an i.d. of 12.7 mm. A feed gas containing 5 vol % propylene, 20 vol % steam and 12% oxygen was passed at a volume hourly space velocity of 1000—1200 $hr^{-1}$ over the catalyst. The pressure in the catalyst was about 1.76 bar (1.76 $kg/cm^2$) gauge. Analysis by gas chromatography of the effluent gases gave the results designated in Tests 3—6 of Table A.

TABLE A

| Test No. | Time, hrs. | Temp. °C | Conversion of propylene, % | Selectivity to acrolein and acrylic acid, % |
|---|---|---|---|---|
| 3 | 78.0 | 310 | 91.1 | 87.4 |
| 4 | 81.0 | 310 | 90.6 | 86.3 |
| 5 | 93.5 | 310 | 90.5 | 87.7 |
| 6 | 99.5 | 310 | 90.1 | 89.7 |
| 7 | 102 | 310 | 94.2 | 86.6 |
| 8 | 115 | 309 | 92.6 | 88.7 |
| 9 | 124 | 309 | 92.5 | 87.9 |
| 10 | 139 | 309 | 89.3 | 87.2 |
| 11 | 147 | 309 | 91.4 | 85.6 |
| 12 | 156 | 310 | 93.7 | 86.7 |
| 13 | 180 | 309 | 92.9 | 86.9 |

After the performance of the catalyst had been established with only propylene feed, hydrogen was added to the feed gas to simulate in part the conditions which would pertain to the oxidation reaction when supplied with propylene produced by dehydrogenation of propane. An additional 4 vol % of hydrogen was included with the mixture previously fed. The results are shown as Tests 7—10 in Table A. It will be seen that substantially the same results were achieved as in Tests 3—6. It was concluded that essentially no hydrogen was being oxidized, judging from the operating conditions in the reactor.

The hydrogen was discontinued and the oxidation reaction continued with 4 vol % propane added. The results are shown as tests 11—13 in Table A. Again substantially the same results were obtained as in Tests 3—6, indicating that propane was not affecting the oxidation of propylene and was not itself being oxidized.

These results show that propylene can be oxidized in the presence of hydrogen and propane, the two principal components of the dehydrogenation of propane to propylene. It may be concluded that the integration of the dehydrogenation and oxidation reaction is feasible.

Example 3

Oxidation of acrolein to acrylic acid

To illustrate the oxidation of acrolein to acrylic acid and the effect of hydrogen and propane additions to the feed gas, experiments were carried out which parallel those of Example 2.

A catalyst was prepared corresponding to the composition of Example 1 of U.S. 3,954,855 and according to the procedure described there. The catalyst composition was

$$Mo_{12}V_{4.8}Sr_{0.5}W_{2.4}Cu_{2.2}O_x.$$

A 73 cc sample of the catalyst was 4.7 mm pellets was placed in a tubular reactor having 12.7 mm i.d. The reactor was surrounded by a bath thermostatically providing a uniform predetermined temperature. The feed to the reactor was initially 6—8 vol % acrolein, 51 vol % air, and 45 vol % steam. The volumetric space velocity was 3000 $hr^{-1}$ and the average reactor pressure slightly above atmospheric. The results are shown in Table B.

TABLE B

| Test No. | Time, hrs. | Temp. °C | Conversion of acrolein, % | Selectivity to acrylic acid, % |
|---|---|---|---|---|
| 14 | 126 | 254 | 92.8 | 89.1 |
| 15 | 126 | 251 | 84.5 | 89.2 |
| 16 | 41 | 252 | 86.8 | 88.4 |
| 17 | 36 | 251 | 80.7 | 87 |

As was done in Example 2, first 4 vol % hydrogen was added and then the hydrogen was replaced with propane. The results are shown in Table B as Test 16 ($H_2$) and 17 (propane). Although some difference in the conversion of acrolein appears, it is clear from Tests 14 and 15 that conversion is very sensitive to temperature and the differences among Tests 15, 16, and 17 are not considered significant. The selectivity to acrylic acid is substantially the same. Again, it may be concluded that an integrated process is feasible.

Separation of acrylic acid (16) may be done by methods known to the prior art. See for example, U.S. 3,926,744. The acrylic acid reactor effluent may be quenched with water or a high-boiling point solvent. Acrylic acid can be removed from the aqueous solution by solvent extraction with a suitable liquid, e.g. ethyl acetate. The resulting acrylic acid-solvent solutions may be separated and purified by distillation to produce acrylic acid.

In its broadest form, the invention includes the integration of dehydrogenation and two oxidation stages whereby the propane is converted to acrylic acid, after which unreacted propane is recycled. Such a combination process without an intermediate separation of propylene has been shown to be possible. If the resulting by-product stream containing significant quantities of propylene and propane can be used for other purposes, then recycling of the gas is not required. In many cases, it will be desirable to recycle unreacted propylene and propane so that the integrated process substantially converts propane to acrylic acid with only minor amounts of by-products and without ever producing a substantially pure propylene stream. In order to recycle gas containing large amounts of propane, it will be necessary to remove hydrogen produced in the dehydrogenation step, excess oxygen from the oxidation step, and carbon oxides formed in all three steps. In addition, a purge of light and heavy by-products and feed impurities will be taken. Removal of carbon dioxide typically would be done by scrubbing all or a portion of the recycle stream with a carbonate or amine solution as is known in the art in order to maintain the desired level of carbon dioxide. Carbon monoxide will be converted to carbon dioxide in the dehydrogenation reactor. Since the presence of these materials is not critical to either the dehydrogenation or oxidation steps, they may be economically permitted to build up in the recycle stream to a level in which they may be conveniently and economically removed. Since the light and heavy hydrocarbon by-products, such as methane, ethane, ethylene, butane, and butenes boil at temperatures significantly different from those of acrolein or the $C_3$ hydrocarbons, they may be separated by distillation. Alternatively, streams containing the by-products in concentrated amounts may be purged. Separation of the hydrogen formed by the dehydrogenation of propane might be done by various methods such as catalytic oxidation, liquid phase absorption or gas phase adsorption.

Figure 2 is a simplified flowsheet showing a preferred embodiment of the invention with a complete process whereby propane is converted substantially to acrylic acid. Fresh propane feed 20 is combined with recycle stream 24 containing principally propane and vaporized in exchanger 22. The combined stream 26 is then heated in exchanger 28 to a temperature such that when combined with the required

amount of steam (30) the temperature obtained will be that suited for the dehydrogenation of propane to propylene. The amount of steam used preferably is suitable for the subsequent oxidation of the propylene to acrolein.

As shown here, steam is supplied as a fresh feed stream. If this is done, then water produced in the reactions is eventually purged from the system from the bottom of the stripper 56. A feed stream containing propane and steam in molar ratios between 1/0.5 and 1/10, preferably 1/1 to 1/5, is fed at temperatures in the range 400—700°C, particularly about 600°C, and at pressures in the range 0—10 bar (0—10 kg/cm$^2$) gauge, preferably near 0 bar (0 kg/cm$^2$) gauge, to reactor 32 where about 50% of the propane is converted to propylene, with a selectivity of 75% or more.

A number of catalysts have been disclosed in the prior art for use in this process and the conditions under which the reaction is carried out will depend on the catalyst selected. Of particular usefulness is a platinum-based catalyst of the type shown in U.S. 4,005,985. Although platinum and tin disposed on a zinc aluminate support provides good performance, other catalysts which have been found effective include platinum and other promoters, such as rhenium and indium supported on zinc aluminate. Other Group VIII noble metals, alone or in combination on various supports known to the art may have application in the dehydrogenation of propane to propylene.

Other potential supports would include alumina, other alkaline earth metal aluminates, and rare earth aluminates including lanthanum. Promoters such as tin, lead, antimony, and thallium may be used. Base metal catalysts such as the chromium, zirconium, titanium, magnesium and vanadium oxides as shown in U.S. 3,479,416 and 3,784,483 or the zinc titanate of U.S. 4,176,140 and 4,144,277 also might be used. The invention is not considered to be limited to specific catalyst formulations.

It will be understood by those skilled in the art that in this process the catalyst rapidly deactivates and must be regenerated frequently. Typically, the process will be operated with multiple reactors so that frequent regeneration is possible. The details of such operations are, however, not considered part of the invention. The dehydrogenation reaction is endothermic and the temperature leaving the reactor 32 will be on the order of 50—200°C lower than the inlet temperature. This will be affected by the amount of steam employed, the condition of the catalyst, and the severity of the reaction conditions chosen.

The dehydrogenation reactor effluent is cooled in exchanger 34 to a suitable temperature for inlet to the oxidation reactor 38 and joined with an oxygen-containing stream 36 to provide a suitable feed for the oxidation of propylene to acrolein. Air enriched in oxygen or air alone might be used, if economic to do so. Although not shown in this flowsheet, it is also possible to cool the effluent from reactor 32 until water is condensed from the gases. Removal of the water would permit adjustment of the steam to propylene ratio used in oxidation reactor 38. The reaction would be carried out under conditions typical of the art, that is, temperatures in the range of about 200—600°C, usually about 300—350°C, pressures of about 0—5 bar (0—5 kg/cm$^2$) gauge, and gas hourly space velocities on the order of 2000 hrs$^{-1}$. A suitable oxidation catalyst will be used, typically a mixture of base metal oxides, especially those which are molybdenum-based. The reactor typically will be of the tubular type where the pelleted catalyst is placed inside tubes which are surrounded by a heat transfer fluid for the removal of the heat of reaction. Typically, 90—95% of the propylene feed to the reactor will be converted to acrolein, along with minor amounts of acrylic acid, acetic acid, and less significant quantities of lighter and heavier by-products. Some of the propylene is burned to carbon oxides and water.

The effluent from reactor 38 is fed with or without intermediate heat exchange and adjustment of the gas composition to reactor 40, where the acrolein is converted to acrylic acid. The reaction would be carried out under conditions typical of the art, that is, temperatures in the range of about 200—600°C, usually about 250—300°C, pressures of about 0—5 bar (0—5 kg/cm$^2$) gauge, and gas hourly space velocities on the order of 2000 hrs$^{-1}$. A suitable oxidation catalyst will be used, typically a mixture of base metal oxides, especially those which are molybdenum-based. The reactor configuration will be similar to that of reactor 38. Typically 90—98% of the acrolein fed to the reactor 40 will be converted to acrylic acid, and minor amounts of acetic acid, carbon oxides, and other by-products. Overall, the selectivity of propylene to acrylic acid would be about 75—80%.

The resulting gaseous mixture may be cooled and fed to a quench tower 42 where an acrylic acid-water mixture is condensed in a recirculating aqueous stream 44 at temperatures in the range of about 30—50°C. Substantially all of the acrylic acid is recovered in a solution containing up to about 35 mol % acrylic acid. This solution is sent to an extraction column 46 where the acrylic acid is selectively extracted by a recirculating stream of a suitable solvent, such as ethyl acetate. The solvent rich with acrylic acid is passed to a stripper 48 to recover the solvent for recycle via line 50. Acrylic acid is passed via line 51 to two additional columns 52 and 54 for separation of the lighter and heavier impurities. Water produced in the process is removed from the bottom of column 46 via stream 47 and sent to stripper 56 for removal of hydrocarbons and then disposed of or recirculated as steam to the dehydrogenation step.

The light gases leaving the top of the quench tower 42 contain large quantities of propane along with lesser amounts of propylene, carbon oxides, hydrogen, oxygen, nitrogen, and light impurities. These gases are passed to absorber 58 for recovery of the $C_3$ content if they are to be returned for further conversion to acrylic acid. Suitable solvents which may be employed include paraffin or aromatic hydrocarbons. Light gases are vented from the process from the top of tower 58. The solvent rich in $C_3$'s is passed to column 60 where they are stripped out and returned to reactor 20 via line 24.

In an alternative embodiment, the gases from absorber 42 could be passed to a reactor where the oxygen content is consumed by reaction with the hydrogen present. This selective oxidation may be carried out over suitable catalysts, such as platinum on alumina, which can carry out the oxidation at a sufficiently low temperature that the valuable $C_3$ hydrocarbons are not oxidized and lost. Once the oxygen has been consumed the gas may be returned to the dehydrogenation reactor, preferably after a slip stream having been scrubbed to remove the carbon dioxide produced by the oxidation steps and to separate $C_3$'s from light by-products, which are purged.

An example of the practical operation of the flow sheet shown in Figure 2 is as follows:

Example 4

One hundred mols/hr of a feed stream 20 containing 99% propane is vaporized in exchanger 22 and fed to the dehydrogenation reactor 32, along with 240 mols/hr of a recycle propane stream 24. The combined streams 26 are heated to about 675°C (28) and mixed with 560 mol/hr of steam (30) which may be provided by recycling and vaporizing stream 49 from the stripper 56. The combined stream 29 is fed to the dehydrogenation reactor 32, where about 29% of the propane fed is converted with 80% selectivity to propylene over a promoted platinum catalyst. Leaving the reactor 32 at about 600°C, the effluent stream is cooled to about 125°C in exchanger 34 and mixed with 152 mol/hr of oxygen (36) before being supplied to the oxidation reactor 38, where about 93% of the propylene is converted with 88% selectivity to acrolein over a promoted molybdenum catalyst. Leaving the reactor 38 at about 325°C and 1.4 bar (1.4 kg/$cm^2$) gauge the effluent gases are passed to the second oxidation reactor 40 for conversion of acrolein to acrylic acid. Leaving the reactor (40) at about 270°C and 1.0 bar (1.0 kg/$cm^2$) gauge the effluent steam is cooled to about 140°C and enters quench tower 42 where water, acrolein, and acrylic acid are condensed and absorbed in a recirculating stream 44, cooled to about 35°C in exchanger 43.

The crude recycle gas leaving the top of the absorber 42 is passed to absorber 58 where it is contacted with a recirculating stream of 250 mol/hr containing $C_8$ paraffins to recover substantially all of the $C_3$ content. The waste gases, i.e. 250 mol/hr of hydrogen, oxygen, nitrogen, and light by-products, are vented from the top of column 58. The enriched liquid is stripped in column 60 and 240 mol/hr of propane is recovered and recycled via line 24 to dehydrogenation reactor 32.

An aqueous stream of 550 mol/hr containing 12 mol % acrylic acid, along with acrolein and miscellaneous by-products such as acetic acid, maleic acid, formic acid, and other oxygenated compounds is passed to extractor 46 where it passes countercurrently versus 190 mol/hr lean solvent (ethyl acetate) entering the base of the tower via line 50. Substantially all of the acrylic acid is absorbed. Water raffinate is withdrawn from the bottom of column 46 and passed via line 47 to column 56 where light by-products are distilled overhead and withdrawn as a waste stream. Stripped water is withdrawn (49) from the bottom of the column for reuse or disposal. The enriched solvent passes overhead from column 46 and is distilled in column 48 to separate the solvent which is recovered overhead and recirculated to column 46 via line 50. The crude acrylic acid leaves the bottom of column 48 and is sent to column 52 via line 51.

The crude acrylic acid stream of 70 mol/hr comprises 90 mol % acrylic acid, with the remainder acetic acid, ethyl acetate and heavy by-products. Light impurities and any residual water are distilled overhead and passed via line 53 to column 56. Partially purified acrylic acid is sent to column 54 via line 55 where it is distilled away from the heavy by-products and withdrawn as a sidestream. High boiling waste materials such as maleic acid are withdrawn from the bottom of column 54 for disposal. Any residual light materials are sent to column 56.

Figure 3 is a simplified flow sheet showing an alternative embodiment of the invention where acrolein is recovered and purified from the effluent of reactor 38 before being oxidized to acrylic acid in reactor 40. The previous discussion of the dehydrogenation and first oxidation steps is still pertinent. In this embodiment, the effluent from reactor 38 is cooled and then led to a quench tower 39A where acrolein is scrubbed from the gases by a recirculating aqueous stream. Gases which are not absorbed may be purged or preferably, the $C_3$ content of the gases is separated by absorption or the like and returned to reactor 32 via line 24. After being absorbed in tower 39A, the enriched absorbent is stripped in tower 39B to provide stream rich in acrolein to the second oxidation reactor 40 via line 39C. Air or oxygen is added and the oxidation to acrylic acid is carried out as previously described. Recovery of the acrylic acid from the reactor effluent proceeds as in Figure 2, as does the purification of acrylic acid.

An example of the operation of the process shown in Figure 3 is as follows:

Example 5

One hundred mols/hr of a feed stream 20 containing 99% propane is dehydrogenated to propylene and then oxidized to acrolein under the conditions of Example 4 except that upon leaving reactor 38 the effluent is cooled to 120°C and fed to absorber 39A, where the acrolein is scrubbed out by countercurrent contact with water. The rich absorbent is stripped in column 39B by vapor supplied by the reboiler. The light gases are returned to the dehydrogenation reactor 32 while 64 mol/hr of acrolein, along with minor amounts of by-products are sent via line 39C to the second oxidation reactor 40. The remainder of the flow sheet is substantially the same as that shown in Figure 2.

Although in its principal aspects the invention is directed to the preparation of acrylic acid from propane, it should be understood that acrolein can be recovered from the effluent of the first oxidation

reactor, as shown in Figure 3 for example, and used as a product of the process where acrolein is needed for various end uses, such as preparation of allyl alcohol.

**Claims**

1. A process for the preparation of acrylic acid from propane comprising:

(a) in a first stage dehydrogenating propane to propylene, optionally in the presence of steam, over a dehydrogenation catalyst to form an effluent stream comprising propylene, hydrogen, carbon oxides and unreacted propane;

(b) in a second stage mixing oxygen and optionally steam with said effluent stream of (a) and passing the mixture over a first oxidation catalyst at conditions selected to produce a second effluent stream comprising acrolein, unreacted propylene and propane, and oxygen, hydrogen and carbon oxides;

(c) in a third stage passing the effluent stream of (b) optionally with additional oxygen over a second oxidation catalyst at conditions selected to produce a third effluent stream comprising acrylic acid, unreacted propylene, propane and acrolein, and oxygen, hydrogen, and carbon oxides;

(d) recovering the acrylic acid from said effluent of (c) to produce an acrylic acid-depleted effluent; and

(e) returning at least the unreacted propane from said acrylic acid-depleted effluent to step (a).

2. The process of claim 1 wherein in said dehydrogenation stage (a) the ratio of propane to steam is in the range 1/0.5 to 1/10 the temperature is in the range 400—700°C and the pressure is up to 10 kg/cm$^2$ gauge.

3. The process of claim 1 or claim 2 wherein said dehydrogenation stage (a) employs a catalyst comprising a Group VIII noble metal or a base metal oxide on a support.

4. A process as claimed in any one of claims 1 to 3 characterized in that the effluent stream of (a) is cooled and water is condensed and separated therefrom before said effluent stream enters the second stage of (b).

5. A process as claimed in any one of claims 1 to 4 characterized in that said effluent of (c) is cooled to condense water therefrom and acrylic acid is recovered as an aqueous solution, thereby forming a residual effluent gaseous stream comprising unreacted propylene, propane and acrolein, and oxygen and carbon oxides.

6. A process as claimed in claim 5 characterized in that the propylene, propane and acrolein of said residual gaseous stream are absorbed in a suitable solvent and thereafter recovered from said solvent and returned to step (a).

7. A process as claimed in claim 5 characterized in that said residual gaseous stream is passed over a suitable catalyst at selective oxidation conditions for removal of said oxygen by reaction with hydrogen and thereafter returned to step (a).

8. A process as claimed in any one of claims 1 to 7 characterized in that the effluent stream of step (b) is treated to increase the acrolein content of said effluent stream prior to passing it over said second oxidation catalyst.

**Patentansprüche**

1. Verfahren zur Herstellung von Acrylsäure aus Propan, dadurch gekennzeichnet, daß man

(a) in einer ersten Stufe Propan durch Dehydrierung, gegebenenfalls in Gegenwart von Wasserdampf, über einen Dehydrierungskatalysator in Propylen überführt und so einen Reaktionsabstrom bildet, der Propylen, Wasserstoff, Kohlenoxide und nichtumgesetztes Propan enthält,

(b) den gemäß Stufe (a) erhaltenen Reaktionsabstrom in einer zweiten Stufe mit Sauerstoff und gegebenenfalls Wasserdampf vermischt und das Gemisch unter solchen Bedingungen über einen ersten Oxidationskatalysator leitet, daß hierdurch ein zweiter Reaktionsabstrom gebildet wird, der Acrolein, nichtumgesetztes Propylen und Propan sowie Sauerstoff, Wasserstoff und Kohlenoxide enthält,

(c) den nach Stufe (b) erhaltenen Reaktionsabstrom in einer dritten Stufe gegebenenfalls mit weiterem Sauerstoff unter solchen Bedingungen über einen zweiten Oxidationskatalysator leitet, daß hierdurch ein dritter Reaktionsabstrom gebildet wird, der Acrylsäure, nichtumgesetztes Propylen, Propan und Acrolein sowie Sauerstoff, Wasserstoff und Kohlenoxide enthält,

(d) aus dem gemäß Stufe (c) erhaltenen Reaktionsabstrom die Acrylsäure gewinnt und so einen an Acrylsäure erschöpften Reaktionsabstrom erzeugt und

(e) wenigstens das in dem an Acrylsäure erschöpften Reaktionsabstrom vorhandene nichtumgesetzte Propan in die Stufe (a) rückführt.

2. Verfahren nach Anspruch 1, dadurch gekennzeichnet, daß man in der Stufe (a) der Dehydrierung bei einem Verhältnis von Propan zu Wasserdampf im Bereich von 1:0,5 bis 1:10, einer Temperatur im Bereich von 400 bis 700°C und einem Überdruck von bis zu etwa 10 bar arbeitet.

3. Verfahren nach Anspruch 1 oder 2, dadurch gekennzeichnet, daß man in der Stufe (a) der Dehydrierung einen Dehydrierungskatalysator verwendet, der ein Edelmetall aus der Gruppe VIII des Periodensystems der Elemente oder ein Grundmetalloxid auf einem Träger enthält.

4. Verfahren nach irgendeinem der Ansprüche 1 bis 3, dadurch gekennzeichnet, daß man den

Reaktionsabstrom der Stufe (a) abkühlt und hierdurch das in ihm enthaltene Wasser kondensiert und davon abtrennt bevor dieser Reaktionsabstrom der zweiten Stufe (b) zugeführt wird.

5. Verfahren nach irgendeinem der Ansprüche 1 bis 4, dadurch gekennzeichnet, daß man den Reaktionsabstrom der Stufe (c) zur Kondensation des Wassers abkühlt und die Acrylsäure als wäßrige Lösung gewinnt und so einen restlichen gasförmigen Reaktionsabstrom bildet, der nichtumgesetztes Propylen, Propan und Acrolein sowie Sauerstoff und Kohlenoxide enthält.

6. Verfahren nach Anspruch 5, dadurch gekennzeichnet, daß man das Propylen, Propan und Acrolein aus dem restlichen gasförmigen Reaktionsabstrom in einem geeigneten Lösungsmittel absorbiert und diese Produkte dann aus diesem Lösungsmittel gewinnt und in die Stufe (a) rückführt.

7. Verfahren nach Anspruch 5, dadurch gekennzeichnet, daß man den restlichen gasförmigen Reaktionsabstrom unter solchen selektiven Oxidationsbedingungen über einen geeigneten Katalysator leitet, daß hierdurch der Sauerstoff durch Reaktion mit Wasserstoff entfernt wird, und diesen Strom dann in die Stufe (a) rückführt.

8. Verfahren nach irgendeinem der Ansprüche 1 bis 7, dadurch gekennzeichnet, daß man den Reaktionsabstrom der Stufe (b) einer Behandlung zur Erhöhung des in diesem Reaktionsabstrom vorhandenen Gehalts an Acrolein unterzieht, bevor man diesen Reaktionsabstrom über den zweiten Oxidationskatalysator leitet.

**Revendications**

1. Procédé de préparation de l'acide acrylique à partir du propane, comprenant:

(a) dans une première étape, la déshydrogénation du propane en propylène, éventuellement en présence de vapeur d'eau, sur un catalyseur de déshydrogénation pour former une courant effluent comprenant du propylène, de l'hydrogène, des oxydes de carbone et du propane n'ayant pas réagi;

(b) dans une seconde étape, le mélangeage de l'oxygène et éventuellement de la vapeur d'eau audit courant effluent provenant de (a) et le passage du mélange sur un premier catalyseur d'oxydation, dans des conditions choisies pour produire un second courant effluent comprenant de l'acroléine, du propylène et du propane n'ayant pas réagi, et de l'oxygène de l'hydrogène et des oxydes de carbone;

(c) dans une troisième étape, le passage du courant effluent provenant de (b), éventuellement avec un supplément d'oxygène, sur un second catalyseur d'oxydation dans des conditions choisies pour produire un troisième courant effluent comprenant de l'acide acrylique, du propylène, du propane et de l'acroléine n'ayant pas réagi, et de l'oxygène, de l'hydrogène et les oxydes de carbone;

(d) la récupération de l'acide acrylique à partir dudit effluent provenant (c) pour produire un effluent appauvri en acide acrylique; et

(e) le renvoi d'au moins le propane, n'ayant pas réagi, provenant dudit effluent appauvri en acide acrylique, vers l'étape (a).

2. Procédé selon la revendication 1, dans lequel, dans ladite étape (a) de déshydrogénation, le rapport du propane, à la vapeur d'eau se situe dans l'intervalle allant de 1/0,5 à 1/10, la température se situe dans l'intervalle allant de 400 à 700°C et la pression manométrique va jusqu'à 10 kg/cm$^2$.

3. Procédé selon la revendication 1 ou la revendication 2, dans lequel ladite étape (a) de déshydrogénation utilise un catalyseur comprenant un métal noble du groupe VIII ou un oxyde de métal de base sur un support.

4. Procédé selon l'une quelconque des revendications 1 à 3, caractérisé en ce que le courant effluent provenant de (a) est refroidi et l'eau est condensée et séparée de ce courant avant que ledit courant effluent n'entre dans la seconde étape (b).

5. Procédé selon l'une quelconque des revendications 1 à 4, caractérisé en ce que ledit effluent provenant de (c) est refroidi pour condenser l'eau, et l'acide acrylique est récupéré sous forme d'une solution aqueuse, ce qui forme un courant gazeux effluent résiduel comprenant du propylène, du propane et de l'acroléine n'ayant pas réagi, ainsi que de l'oxygène et des oxydes de carbone.

6. Procédé selon la revendication 5, caractérisé en ce que le propylène, le propane et l'acroléine dudit courant gazeux résiduel sont absorbés dans un solvant convenable, puis récupérés à partir de ce solvant et renvoyés vers l'étape (a).

7. Procédé selon la revendication 5, caractérisé en ce que ledit courant gazeux résiduel est envoyé sur un catalyseur convenable dans des conditions d'oxydation sélective pour l'enlèvement dudit oxygène par réaction avec l'hydrogène, puis il est renvoyé vers l'étape (a).

8. Procédé selon l'une quelconque des revendications 1 à 7, caractérisé en ce que le courant effluent provenant de l'étape (b) est traité pour en augmenter la teneur en acroléine avant d'être envoyé sur ledit second catalyseur d'oxydation.

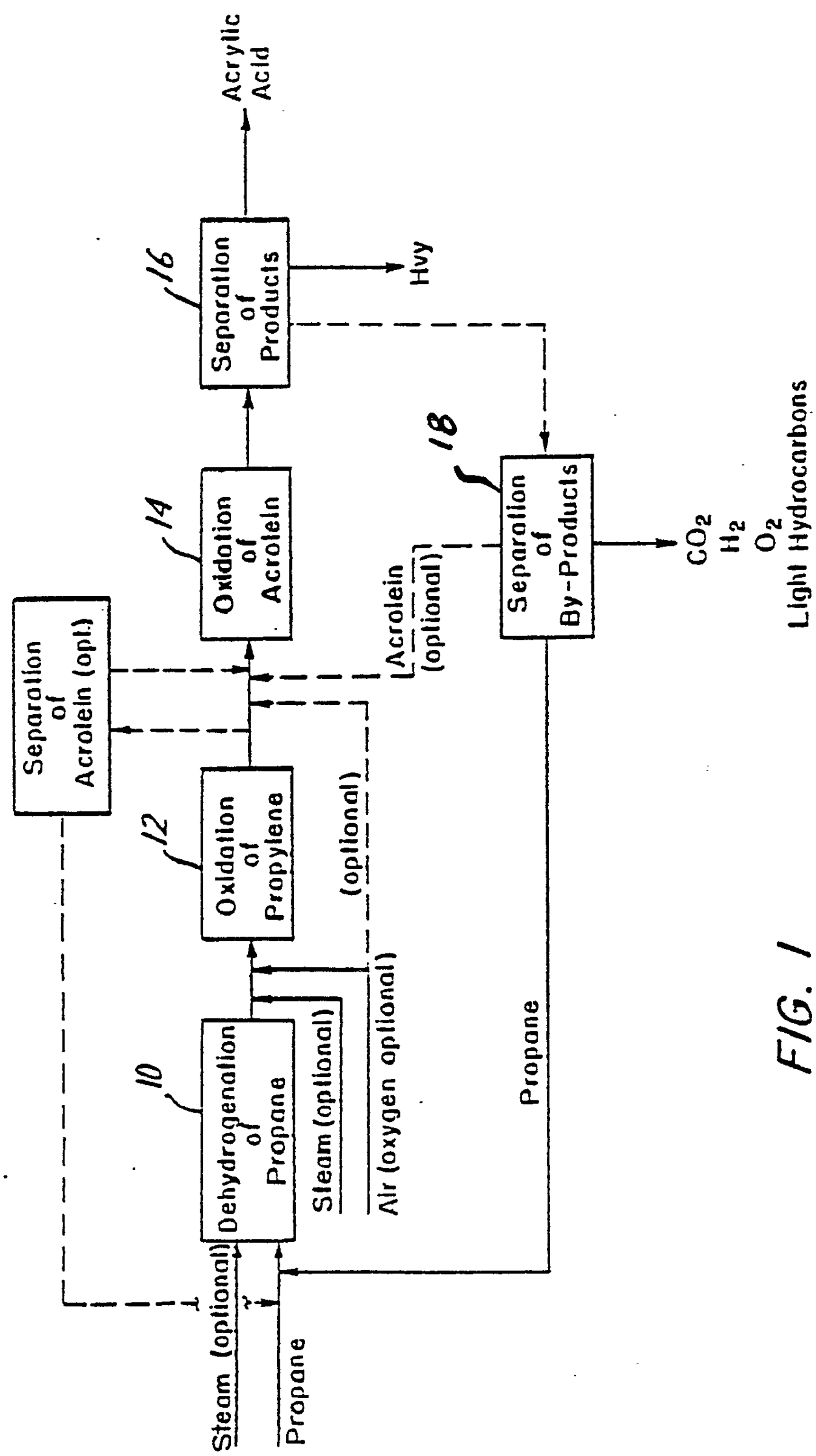

Acrylic Acid
16
Separation of Products
Hvy
18
Separation of By-Products
CO2
H2
O2
Light Hydrocarbons
14
Oxidation of Acrolein
Acrolein (optional)
Separation of Acrolein (opt.)
12
Oxidation of Propylene
(optional)
10
Dehydrogenation of Propane
Steam (optional)
Air (oxygen optional)
Propane
Steam (optional)
Propane


FIG. 1

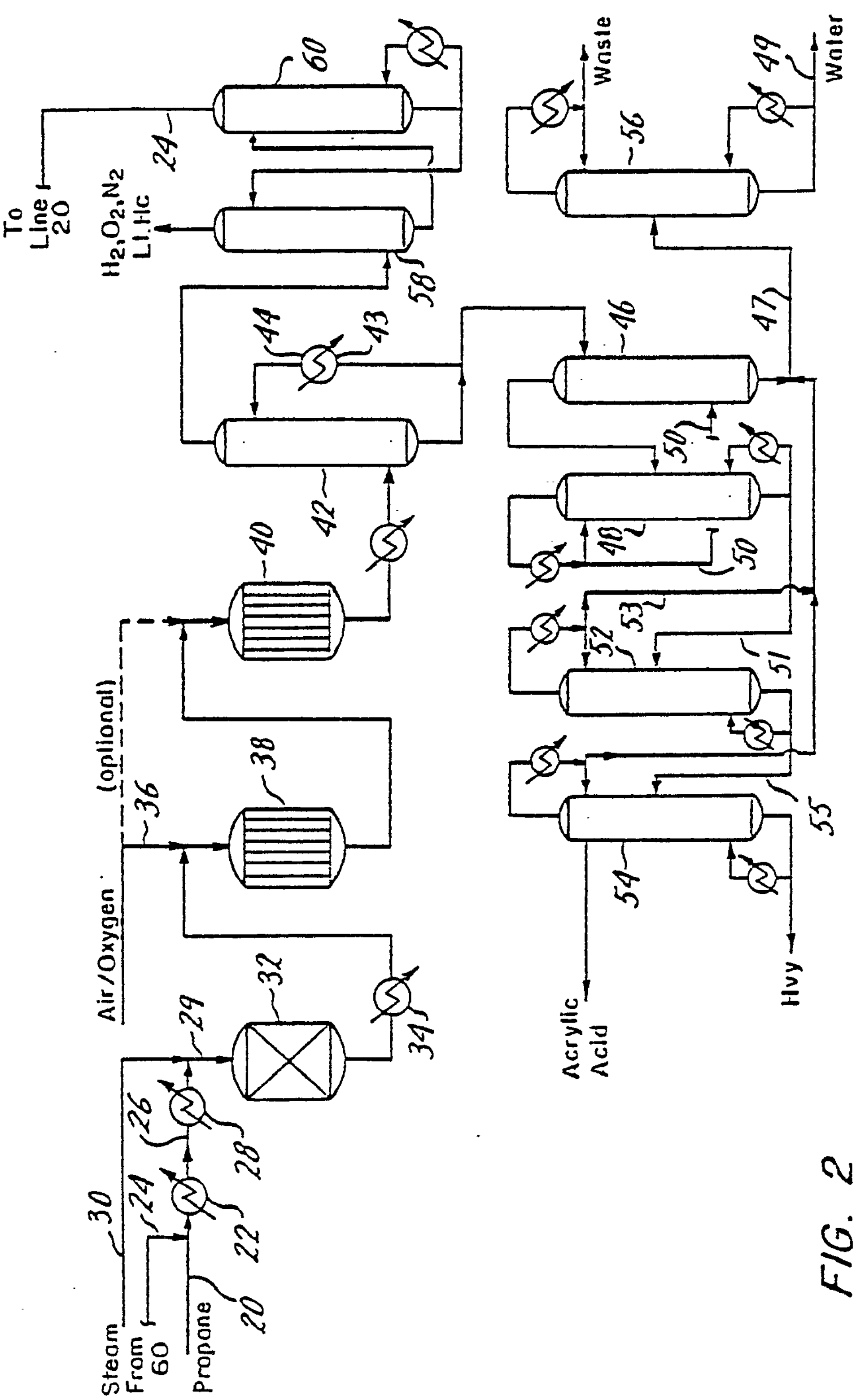
Steam
From 60
Propane
30
24
20
22
26
28
29
32
34
Air/Oxygen
36
(optional)
38
40
42
43
44
To Line 20
24
$H_2,O_2,N_2$ Lt.Hc
58
60
46
47
48
50
51
52
53
54
55
56
49
Waste
Water
Acrylic Acid
Hvy

FIG. 2

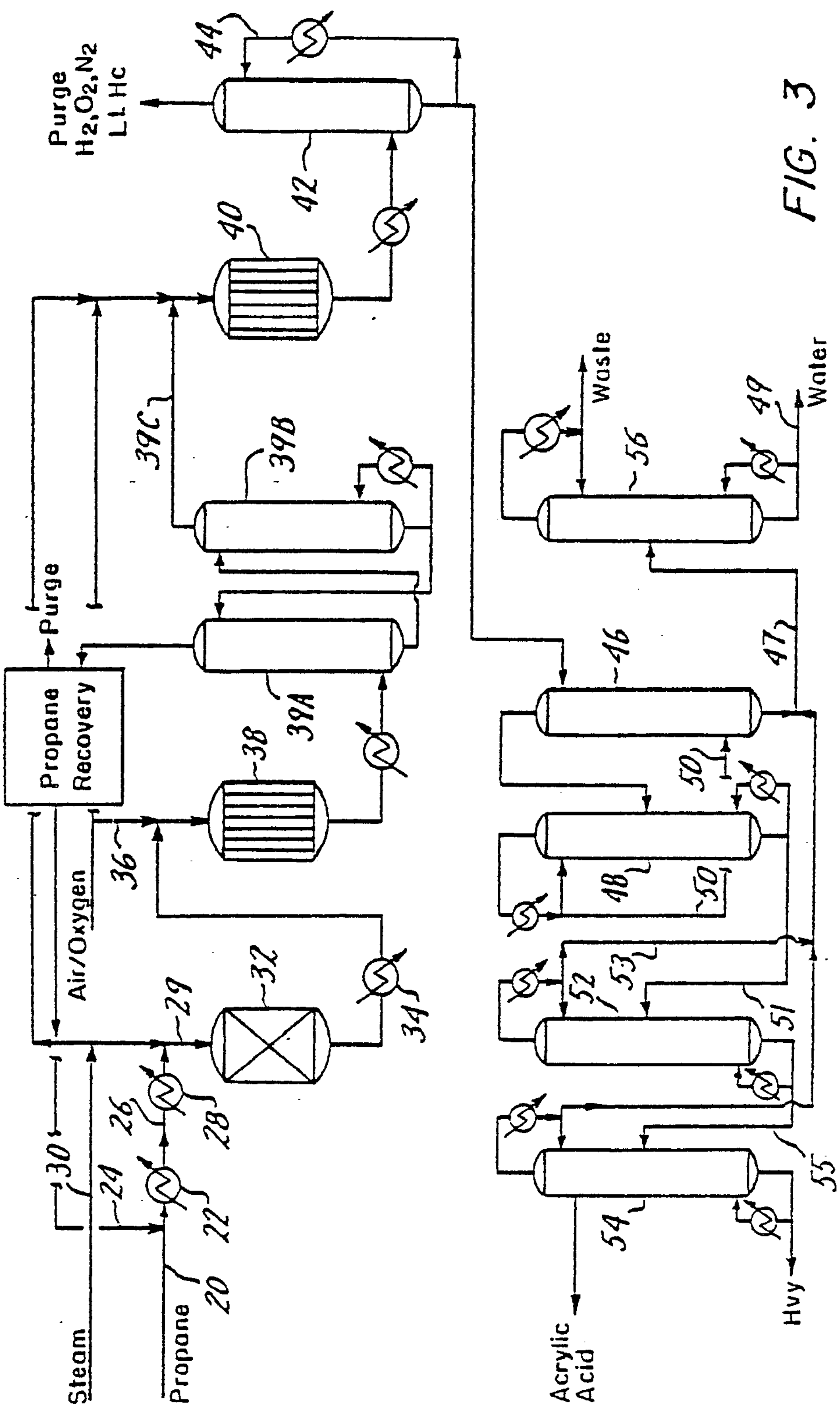
Steam
Propane
Air/Oxygen
Purge
Propane Recovery
Purge
H2,O2,N2
Lt Hc
Waste
Water
Acrylic Acid
Hvy
20
22
24
26
28
29
30
32
34
36
38
39A
39B
39C
40
42
44
46
47
48
49
50
51
52
53
54
55
56

FIG. 3